# FASCICULE DE BREVET EUROPEEN

(11) **EP 2 200 541 B1**
(45) Date de publication et mention de la délivrance du brevet: **26.09.2012**
(21) Numéro de dépôt: 08858898.3
(22) Date de dépôt: 03.10.2008
(51) Int. Cl.: A61F 2/44

(54) **PROTHESE INTERVERTEBRALE**
BANDSCHEIBENPROTHESE
INTERVERTEBRAL PROSTHESIS

(30) Priorité: 05.10.2007 FR 0706987
(43) Date de publication de la demande: 30.06.2010
(73) Titulaire: Warsaw Orthopedic, Inc., Warsaw, IN 46581 (US)
(72) Inventeur: POINTILLART, Vincent, 33000 Bordeaux (FR); Assaker Richard, 7540 Kain (BE)
(74) Mandataire: Viering, Jentschura & Partner
(86) Numéro de dépôt international: PCT/FR2008/001386
(87) Numéro de publication internationale: WO 2009/074756

(56) Documents cités:
- EP-A- 1 532 950
- DE-U1- 20 315 613
- FR-A- 2 775 891
- FR-A- 2 787 017
- FR-A- 2 893 248
- FR-A- 2 894 808
- US-A1- 2007 191 958

## Description

L'invention concerne une prothèse de disque intervertébral destinée à remplacer partiellement ou totalement les disques naturels assurant la liaison entre deux vertèbres de la colonne vertébrale, quelle que soit la partie concernée du rachis.

### ARRIERE PLAN DE L'INVENTION

Les disques intervertébraux de la colonne vertébrale sont constitués chacun d'un élément central appelé nucleus pulposus, enfermé dans un enroulement de fibres appelées annulus. Le disque assure la liaison entre deux corps vertébraux et le contrôle des mouvements de flexion, d'inclinaison et de rotation de la colonne vertébrale. Ce disque est amené à se dégrader sous l'effet du temps, des efforts ou de certaines maladies dégénératives, il en résulte alors un tassement et/ou un mauvais fonctionnement de celui-ci. Ceci peut entraîner différents types de pathologies, occasionnant de multiples douleurs plus ou moins intenses ainsi que des handicaps plus ou moins importants.

Le traitement de ce type d'affection consiste à pratiquer l'ablation du disque malade et son remplacement soit par un élément mobile ou déformable soit par un élément qui relie rigidement les deux vertèbres concernées.

Plusieurs types de prothèses ont été proposés pour remplacer le disque intervertébral mais elles ne sont que partiellement satisfaisantes. En effet, elles préservent la mobilité intervertébrale et restaure la distance intervertébrale à une valeur proche de celle assurée par un disque sain. En revanche elles imposent, dans cette mobilité, une cinématique particulière qui n'est pas compatible ou qui n'est que partiellement compatible avec la mobilité naturelle relative de deux vertèbres. La prothèse, dans la plupart des cas, impose une cinématique qui lui est propre, avec son centre de rotation et ses différents guidages plan sur plan, qui ne manque pas de venir en interférence avec les éléments de l'articulation naturelle qui demeurent entre deux vertèbres, notamment les facettes articulaires postérieures. Il faut noter à cet égard que le soin avec lequel on doit poser la prothèse est important car toute imprécision dans ce placement augmente la gravité du conflit entre la cinématique de la prothèse et la cinématique naturelle. Cette mobilité non physiologique peut être à l'origine de conséquences cliniques indésirables. On peut même craindre des risques de migration d'un composant ou de luxation de l'articulation prothétique.

De plus, pour la plupart d'entre elles, les prothèses connues ne sont pas de nature à restaurer la lordose cervicale ou lombaire normale. La restauration de la distance intervertébrale ne prend pas en compte l'inclinaison nécessaire d'une vertèbre à l'autre dans l'empilement qui conduit à cette lordose dont l'existence est utile à une biomécanique normale de l'ensemble du rachis, et plus particulièrement des étages adjacents.

Par ailleurs, les prothèses connues ne sont pas adaptées à absorber les chocs. La conséquence de cette inaptitude, associée au conflit entre la cinématique naturelle et la cinématique de l'articulation, peut conduire à la production d'une usure prématurée, et des éléments naturels, et des éléments prothétiques, risquant de dégrader l'état clinique du patient.

Une prothèse de disque intervertébral connue notamment par WO-2007057555 comporte :
- un plateau supérieur rigide,
- un plateau inférieur rigide,
- un coussin intermédiaire élastiquement compressible, logé entre les surfaces internes des deux plateaux, l'ensemble ayant la particularité d'être divisé dans le sens de l'épaisseur en deux unités reposant l'une sur l'autre par des surfaces de contact complémentaires. Cette structure se présente sous la forme d'une pièce qui n'impose aux deux vertèbres qu'elle relie aucune liaison contrainte (bien entendu pour des amplitudes correspondant à un déplacement relatif naturel). Il s'ensuit que les guides naturels de ce déplacement relatif restent prépondérants (facettes articulaires postérieures notamment) et leur intégrité est préservée. En outre, au niveau de ces surfaces de contact, il est possible par le choix approprié de leur forme et de leur état, de maîtriser la nature et la concentration des contraintes ainsi que les déformations qui existeront à leur niveau.

### OBJET DE L'INVENTION

La présente invention entend remédier aux inconvénients des prothèses classiques et constituer une prothèse complémentaire aux prothèses décrites dans le document cité tout en en conservant la simplicité de structure et la facilité de pose.

### RESUME DE L'INVENTION

À cet effet, la présente invention a pour objet une prothèse de disque intervertébral qui comporte :
- un plateau supérieur rigide,
- un plateau inférieur rigide,
- un coussin intermédiaire élastiquement compressible, logé entre les deux surfaces internes des plateaux et possédant, au repos, la forme d'un coin avec au moins une entaille avant ouverte du côté le plus large du coin et une entaille arrière ouverte du côté le plus étroit, les fonds d'entaille étant distants l'un de l'autre et situés dans la moitié la plus étroite de la prothèse, chaque entaille étant limitée depuis ce fond par des surfaces s'étendant en regard l'une de l'autre dont les zones en contact sont variables en fonction de l'intensité et de la direction de la charge de compression appliquée au coussin.

Par cette structure, le lien entre deux vertèbres est de deux naturels :
- la partie du coussin laissée intacte entre les entailles, de dimensions réduites ce qui limite considérablement les contraintes pouvant être transmises entre vertèbres lors de leur mouvement relatif tout en assurant une cohésion au moins en compression du coussin,
- les parties entaillées du coussin qui offrent des contacts de grandeurs différentes selon la position des vertèbres l'une par rapport à l'autre et selon la compression qui s'exerce sur ce coussin. Ces parties entaillées constituent le moyen de rendre « variable » le comportement en déformation du coussin en fonction de la position et de la charge des vertèbres concernées. La forme et la nature des faces de ces entailles qui coopèrent par contact sont des facteurs sur lesquels, à la fabrication du coussin, on peut jouer (par exemple par modélisation) afin d'ajuster le comportement du coussin au besoin du patient.

En outre, la forme en coin de la structure permet de restaurer la lordose du rachis que la dégénérescence discale a en général détériorée.

Le coussin est par exemple à base de polyéthylène haute densité, de polyuréthane haute densité, de silicone ou de composites de ces différents matériaux.

Selon une particularité de réalisation, chaque entaille est définie par des faces divergentes de sorte que la surface de contact en jeu dans le coussin est dépendante de l'écrasement de ce dernier, donc de la position et de la charge entre les deux vertèbres concernées.

Dans un mode particulier de l'invention, l'entaille avant possède, entre son débouché (ses bords) et son fond, deux faces en regard définissant des reliefs qui s'imbriquent. Ces reliefs sont des dents interpénétrantes ou des poils ou pics imbriqués. On multiplie ainsi la quantité de surface coopérante et sa variabilité en fonction des critères de position et de charge des vertèbres. Cette géométrie particulière permet également de disposer d'un moyen d'agir sur plus qu'une quantité de surface en contact puisque les parties concernées du coussin ont une épaisseur, du fait de l'imbrication plus ou moins prononcée des reliefs au cours de l'usage de la prothèse. Il s'agit alors des propriétés d'une couche de matériau, de part et d'autre de l'entaille qui sont concernées et variables en fonction du degré d'imbrication.

La prothèse comporte de manière préférée une membrane de contention du coussin fixée aux plateaux. Cette membrane de contention permet d'éviter l'envahissement biologique et la colonisation de la prothèse. Elle assure par ailleurs, bien que tous les matériaux utilisés soient biocompatibles, un isolement complet du coussin à l'égard du milieu biologique voisin et forme une barrière à la migration dans l'organisme d'éventuelles particules provenant du coussin.

Cette membrane peut-être un anneau fixé aux plateaux (laissant un contact direct coussin-plateau), ou bien un sac hermétique, enfermant le coussin, et par exemple collé aux plateaux, ou encore une membrane prise en sandwich entre chaque plateau et une platine rivetée à celui-ci, l'autre face de cette platine étant en contact avec le coussin.

D'autres dispositions constructives apparaîtront à la lecture de ce qui suit. On mentionnera en particulier l'existence d'un lien entre les deux plateaux de la prothèse qui permet de la maintenir dans un état comprimé afin d'en faciliter la pose. L'un des avantages de cette prothèse réside dans la pose aisée de celle-ci qui ne requiert pas une grande précision puisque elle n'impose pas de devoir « accorder » ses degrés de liberté avec ceux, naturels, existant entre deux vertèbres.

### BREVE DESCRIPTION DES DESSINS

D'autres caractéristiques et avantages de l'invention ressortiront de la description donnée ci-après de quelques exemples de sa réalisation.

### DESCRIPTION DETAILLEE DE L'INVENTION

Il sera fait références aux dessins annexés parmi lesquels :
- la figure 1 représente, par une vue schématique en coupe, une prothèse selon l'invention placée entre deux vertèbres cervicales,
- la figure 2 est une vue éclatée, également schématique, de cette prothèse,
- la figure 3 est une vue de la prothèse de la figure 2 refermée et maintenue comprimée par un lien sécable,
- les figures 4 à 6 sont des représentations en coupe antéro-postérieures de trois variantes de réalisation du coussin déformable mis en oeuvre dans la prothèse selon l'invention,
- la figure 7 est une vue en coupe VII-VII de la figure 4,
- la figure 8 illustre par une vue extérieure, une demi-prothèse intervertébrale lombaire,
- la figure 9 étant le schéma illustrant l'implant entre deux corps vertébraux lombaires.

### DESCRIPTION DETAILLEE DE L'INVENTION

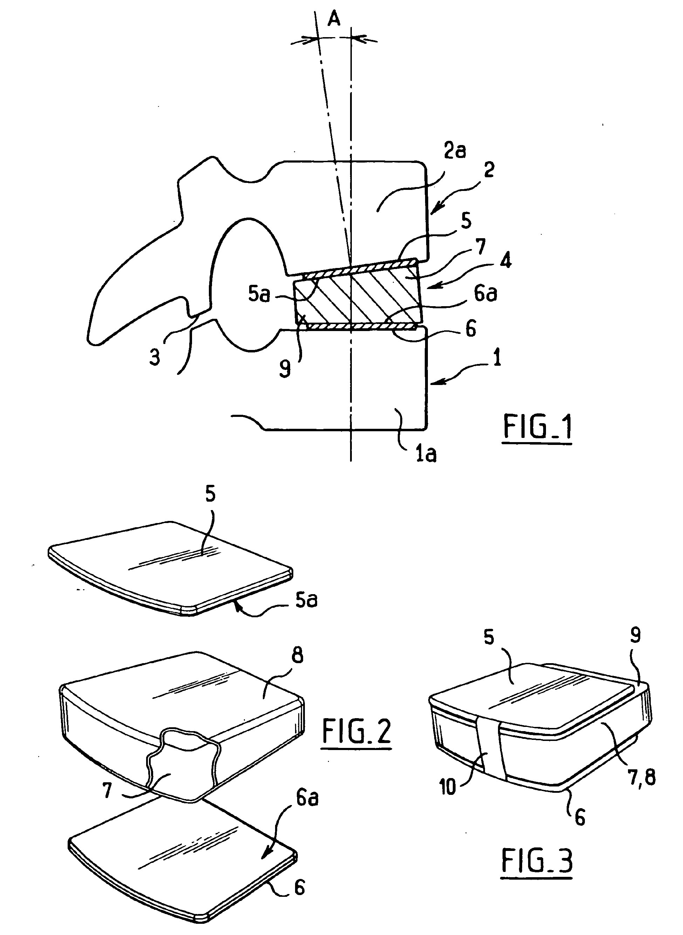

À la figure 1, on a représenté deux vertèbres cervicales successives 1 et 2 avec chacune un corps vertébral la et 2a, et une surface articulaire postérieure 3. Entre les corps vertébraux, on a représenté, de manière schématique, une prothèse 4 qui comporte deux plateaux extrêmes 5 et 6 et un corps intermédiaire 7 en forme de coussin déformable élastiquement, notamment en compression, qui a la forme d'un coin dont le sommet ou l'arête (l'extrémité la moins épaisse) est postérieur, la partie de droite de la figure représentant la zone antérieure du rachis.

Les plateaux 5 et 6 extrêmes de la prothèse sont au contact des corps vertébraux 1a et 2a par l'intermédiaire de surfaces préalablement préparées sur ces corps après ablation du disque naturel détérioré. La surface externe de ces plateaux peut comporter des moyens d'ancrage, par exemple des reliefs, pour améliorer la fixation à l'os ; il peut également s'agir de vis ou tout élément équivalent. On a représenté la lordose du rachis par l'angle A qu'impose la prothèse à l'orientation relative des deux vertèbres.

À la figure 2, on constate que chacun des plateaux 5 et 6 possède une surface interne 5a, 6a, ici représentée plane mais qui peut affecter une forme concave sensiblement sphérique ou cylindrique. Le coussin compressible 7 est fixé aux plateaux. Ce coussin est enfermé dans une membrane de contention 8, qui isole biologiquement ce coussin du reste du corps du patient. La fixation de la membrane au reste de la prothèse sera réalisée par tout moyen approprié. On citera par exemple une membrane « cylindrique » fixée au bord de chaque plateau (par sertissage notamment); la membrane peut également être telle que son bord est pris en sandwich entre la face interne de chaque plateau et une platine rapportée sur cette dernière par exemple par rivetage ; elle peut être en forme de sac entièrement fermé sur lequel sont collés les plateaux.

À la figure 3, on a représenté la prothèse dans son état prête à être insérée entre deux vertèbres, cet état correspondant à une compression du coussin compressible 7 maintenue grâce à un lien 10 disposé sur la face antérieure de la prothèse 4 et qui peut être rompu après mise en place de cette dernière entre les deux corps vertébraux la et 2a. Ce lien peut aussi être disposé de manière à entourer la totalité de la prothèse de sorte que sa rupture et son retrait après coupe si nécessaire, peuvent être réalisés par le chirurgien par voie postérieure.

On remarque sur cette figure que les plateaux 5 et 6 sont de dimensions telles qu'ils sont, dans la partie postérieure de la prothèse, en retrait du coussin, lequel déborde des plateaux par une partie 9 du côté de l'arête du coin.

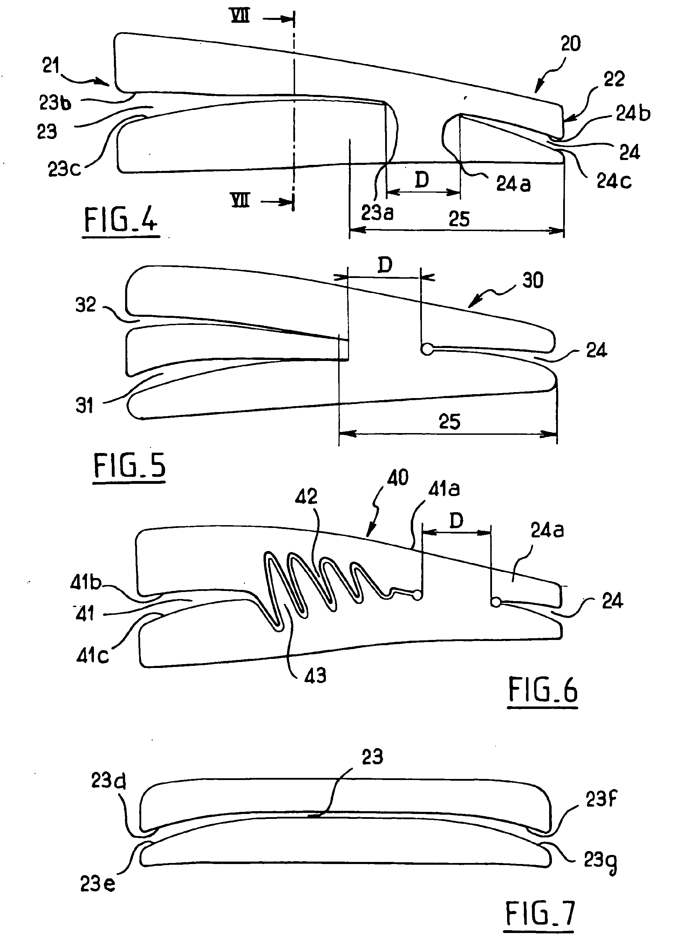

La figure 4 est une vue en coupe d'un premier mode de réalisation 20 du coussin 7 de l'invention. Les plateaux ne sont pas représentés. Ce coussin, comme le coussin 7, est en forme de coin avec sa face antérieure 21 plus large que sa face postérieure 22. Il est pourvu, dans son épaisseur, d'une entaille avant 23 ouverte du côté le plus large du coin et une entaille arrière 24 ouverte du côté le plus étroit, les fonds 23a et 24a d'entaille étant distants l'un de l'autre d'une valeur D et situés dans la moitié 25 la plus étroite de la prothèse c'est-à-dire la moitié postérieure de cette dernière. Chaque entaille est définie par des faces 23b, 23c ; 24b, 24c qui, lorsque le coussin est sans contrainte, sont écartées l'une de l'autre avec une divergence progressive qui s'accentue au voisinage de son débouché sur la face antérieure ou postérieure de la prothèse. Cette géométrie est un moyen d'augmenter ou de diminuer la fermeture de chaque entaille donc la dimension des surfaces en contact en fonction du degré de compression du coussin à son niveau. On dispose par là d'un moyen de détermination du comportement du coussin (écrasement et/ou déformation) sous l'effet de charges ou de mouvements au niveau des vertèbres adjacentes.

A la figure 5, il s'agit de la représentation par une même vue, d'une variante 30 de réalisation de ce coussin dans laquelle on a prévu deux entailles antérieures 31 et 32 au lieu d'une. On retrouve sur cette figure les éléments de la figure précédente avec les mêmes références.

A la figure 6, le coussin 40 possède une fente antérieure 41 qui est d'un profil tourmenté entre ses bords 41b et 41c jusque vers son fond 41a. Ce profil définit, dans le cas de la figure, une série d'indentations 42 ayant pour racines la partie du coussin au-dessus de l'entaille 41 et 43 implantées dans la partie inférieure de ce coussin, imbriquées les unes dans les autres avec plus ou moins de jeu selon la déformation en compression du coussin. On constate qu'ici aussi, cette géométrie constitue un moyen de donner au coussin des lois de variation de son comportement en déformation.

La figure 7 enfin illustre, par une coupe transversale, la forme de l'entaille antérieure du coussin de la figure 4. On constate que cette entaille est, dans ce plan de profil courbe, avec une convexité tournée vers le haut et des lèvres latérales divergentes 23d, 23e et 23f, 23g.

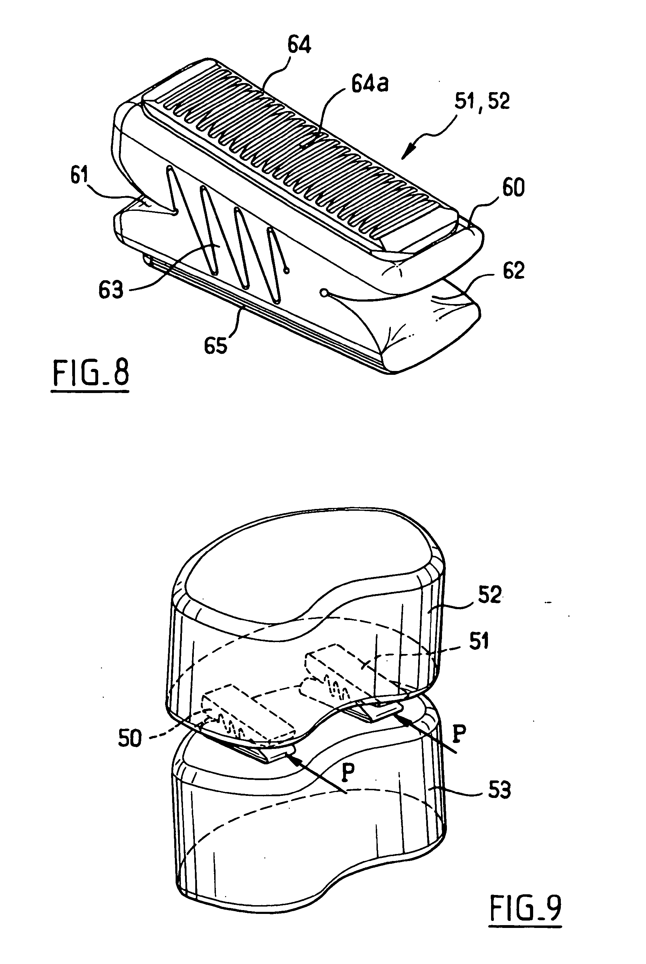

Aux figures 8 et 9, on retrouve la plupart des éléments déjà décrits sous une forme semblable. La prothèse est ici formée de deux demi-prothèses 50 et 51 destinées à être insérées entre deux corps vertébraux lombaires 52 et 53 par voie postérieure, c'est-à-dire dans la direction de la flèche P.

Chacune des demi-prothèses est, conformément à l'invention, composée d'un coussin 60 avec des entailles 61 et 62, des indentations imbriquées 63, une zone de liaison 64 limitée par le fond des entailles 61 et 62 et de plateaux 64, 65, ceux-ci ayant leur surface extérieure pourvue d'une texturation 64a pour favoriser l'ancrage de l'implant dans les corps vertébraux.

L'invention n'est pas limitée à la description qui vient d'être donnée à titre d'exemples. Ainsi, dans une variante non représentée, les indentations sont courbes, par exemple en arc de cercle centré du côté postérieur de l'implant. En outre, les indentations peuvent être remplacées par des poils ou de pics qui eux aussi s'intercalent les uns dans les autres avec plus ou moins de vigueur de sorte que leur résistance au flambement - par exemple - varie en fonction du degré de leur imbrication.

## Revendications

1. Prothèse (4) de disque intervertébral comportant :
- un plateau supérieur rigide (5),
- un plateau inférieur rigide (6),
- un coussin intermédiaire (7) élastiquement déformable, logé entre les deux surfaces internes des plateaux (5,6), **caractérisée en ce que** le coussin (20) possède au repos, la forme d'un coin possédant au moins une entaille avant (23) ouverte du côté le plus large du coin et une entaille arrière (24) ouverte du côté le plus étroit dont les faces coopèrent par contact surfacique d'importance variable en fonction de l'intensité et/ou de la direction de la charge subie par la prothèse, les fonds (23a,24a) d'entaille étant distants (D) l'un de l'autre et situés dans la moitié (25) la plus étroite de la prothèse.

2. Prothèse selon la revendication 1, **caractérisée en ce que** chaque entaille (23,24) possède des faces (23b,23c,24b,24c) divergentes.

3. Prothèse selon l'une des revendications précédentes, **caractérisée en ce que** l'entaille avant (41) possède, entre ses bords (41b,41c) et son fond (41a), deux faces en regard définissant des reliefs (42,43) qui s'imbriquent.

4. Prothèse selon la revendication 3, **caractérisée en ce que** les reliefs sont des dents interpénétrantes.

5. Prothèse selon la revendication 3, **caractérisée en ce que** les reliefs sont des poils ou pics imbriqués.

6. Prothèse selon l'une des revendications précédentes, **caractérisée en ce qu'**elle comporte une membrane (8) de contention du coussin (7) liée aux plateaux (5,6).

7. Prothèse selon l'une des revendications précédentes, **caractérisée en ce qu'**elle comporte un lien (10) de maintien du coussin à l'état comprimé, susceptible d'être sectionné après la mise en place de la prothèse.

8. Prothèse selon la revendication 1, **caractérisée en ce qu'**elle présente deux entailles (31,32) antérieures sensiblement parallèles.

9. Prothèse selon l'une des revendications précédentes, **caractérisée en ce que**, en coupe frontale, au moins l'entaille antérieure (23) possède une courbure générale dont la convexité est tournée vers le haut et présente des lèvres (23d,23e,23f,23g) latérales divergentes.

10. Prothèse selon l'une des revendications précédentes, **caractérisée en ce que**, destinée aux vertèbres lombaires, elle comporte deux demi-prothèses identiques insérables parallèlement entre elles entre deux corps vertébraux adjacents.

## Claims

1. Intervertebral disk prosthesis (4), comprising:
- a rigid upper plate (5),
- a rigid lower plate (6),
- an elastically deformable intermediate cushion (7) housed between the two inner surfaces of the plates (5, 6), **characterized in that** the cushion (20), at rest, has the shape of a wedge having at least a front notch (23) open on the largest side of the wedge and a rear notch (24) open on the narrowest side, the faces of which cooperate by surface contact with a magnitude varying as a function of the intensity and/or the direction of the load to which the prosthesis is subjected, the bottoms (23a, 24a) of the notches being spaced apart (D) from each other and being situated in the narrowest half (25) of the prosthesis.

2. Prosthesis according to claim 1, **characterized in that** each notch (23, 24) has diverging faces (23b, 23c, 24b, 24c).

3. Prosthesis according to any one of the preceding claims, **characterized in that** the front notch (41) has two faces facing each other between its edges (41b, 41c) and its bottom (41a), defining interlocking reliefs (42, 43).

4. Prosthesis according to claim 3, **characterized in that** the reliefs are interpenetrating teeth.

5. Prosthesis according to claim 3, **characterized in that** the reliefs are interlocked bristles or spikes.

6. Prosthesis according to any one of the preceding claims, **characterized in that** it comprises a membrane (8) for holding the cushion (7) connected to the plates (5, 6).

7. Prosthesis according to any one of the preceding claims, **characterized in that** it comprises a link (10) for maintaining the cushion in the compressed state, which is suitable for being cut after the prosthesis has been put into place.

8. Prosthesis according to claim 1, **characterized in that** it comprises two front notches (31, 32) which are substantially in parallel.

9. Prosthesis according to any one of the preceding claims, **characterized in that**, in front section, at least the front notch (23) has a general curvature, the convexity of which is directed upwards, and has diverging lateral lips (23d, 23e, 23f, 23g).

10. Prosthesis according to any one of the preceding claims, **characterized in that**, provided for the lumbar vertebrae, it comprises two identical half-prostheses that can be inserted in parallel to each other between two adjacent vertebral bodies.

## Patentansprüche

1. Bandscheibenprothese (4), aufweisend:
- eine obere steife Platte (5),
- eine untere steife Platte (6),
- ein elastisch verformbares Zwischenpolster (7), das zwischen den beiden Innenflächen der Platten (5, 6) aufgenommen ist, **dadurch gekennzeichnet, dass** das Polster (20) in Ruhestellung die Form eines Keils hat, der aufweist: mindestens eine vordere Einkerbung (23), die auf der breitesten Seite des Keils offen ist, und eine hintere Einkerbung (24), die auf der schmalsten Seite offen ist, wobei ihre Flächen durch Oberflächenkontakt mit variabler Stärke in Abhängigkeit von der Stärke und/oder der Richtung der auf die Prothese aufgebrachten Last zusammenwirken, wobei die Einkerbungsböden (23a, 24a) im Abstand (D) voneinander angeordnet sind und in der schmalsten Hälfte (25) der Prothese angeordnet sind.

2. Prothese gemäß Anspruch 1, **dadurch gekennzeichnet, dass** jede Einkerbung (23, 24) divergierende Flächen (23b, 23c, 24b, 24c) aufweist.

3. Prothese gemäß einem der vorhergehenden Ansprüche,
**dadurch gekennzeichnet, dass** die vordere Einkerbung (41) zwischen ihren Rändern (41b, 41c) und ihrem Boden (41a) zwei einander zugewandte Flächen aufweist, die Reliefs (42, 43) definieren, die ineinander verschachtelt sind.

4. Prothese gemäß Anspruch 3, **dadurch gekennzeichnet, dass** die Reliefs einander durchdringende Zähne sind.

5. Prothese gemäß Anspruch 3, **dadurch gekennzeichnet, dass** die Reliefs ineinander verschachtelte Borsten oder Zacken sind.

6. Prothese gemäß einem der vorhergehenden Ansprüche,
**dadurch gekennzeichnet, dass** sie eine Membran (8) zum Halten des Polsters (7) aufweist, die mit den Platten (5, 6) verbunden ist.

7. Prothese gemäß einem der vorhergehenden Ansprüche,
**dadurch gekennzeichnet, dass** sie ein Verbindungsteil (10) zum Halten des Polsters in dem komprimierten Zustand aufweist, das geeignet ist, nach dem Platzieren der Prothese durchtrennt zu werden.

8. Prothese gemäß Anspruch 1, **dadurch gekennzeichnet, dass** sie zwei im Wesentlichen parallele vordere Einkerbungen (31, 32) aufweist.

9. Prothese gemäß einem der vorhergehenden Ansprüche,
**dadurch gekennzeichnet, dass** mindestens die vordere Einkerbung (23) im Frontschnitt eine allgemeine Krümmung aufweist, deren Wölbung in Richtung nach oben gerichtet ist, und divergierende seitliche Lippen (23d, 23e, 23f, 23g) aufweist.

10. Prothese gemäß einem der vorhergehenden Ansprüche,
**dadurch gekennzeichnet, dass** sie, für die Lendenwirbel bestimmt, zwei identische Halbprothesen aufweist, die parallel zueinander zwischen zwei benachbarte Wirbelkörper einsetzbar sind.
